# EUROPEAN PATENT APPLICATION

(11) **EP 4 505 938 A1**
(43) Date of publication of application: **12.02.2025**
(21) Application number: 24171795.8
(22) Date of filing: 23.04.2024
(51) Int. Cl.: A61B 1/00, A61B 1/015, A61B 1/018, A61B 1/303

(54) **DUAL-CHANNEL INSTRUMENT GUIDING DEVICE FOR HYSTEROSCOPE**

(30) Priority: 09.08.2023 CN 202311001795
(71) Applicant: Liu, Ling, Shanghai 200011 (CN)
(72) Inventor: Liu, Ling, Shanghai 200011 (CN)
(74) Representative: Wächter, Jochen

(57) **Abstract**

Disclosed is a dual-channel instrument guiding device for hysteroscope. The dual-channel instrument guiding device for hysteroscope is provided with a cavity dividing piece provided in an operation sleeve thereof. The cavity dividing piece divides the interior of the operation sleeve into three channels isolated from one another. One of the channels is used as a lens channel for a hysteroscopic lens to pass through, and the remaining two channels are used as instrument channels for surgical instruments to pass through. During actual use, different instruments can be introduced into two instrument channels simultaneously to perform surgical operation on a lesion area in a human body cavity, and the problem that the existing hysteroscope cannot achieve the mutual operation of multiple instruments at the lesion of the cavity simultaneously is solved.

## Description

### TECHNICAL FIELD

The present disclosure relates to the field of medical instruments, and in particular to a dual-channel instrument guiding device for hysteroscope .

### BACKGROUND

Hysteroscopy is a minimally invasive diagnosis and treatment technology, in particular a fiber light source endoscope used for examination and treatment in natural cavities of the body such as vagina and uterine cavity. The front of the endoscope enters the natural cavities such as vagina, uterine cavity, so as to magnify the observed parts, which can visually and accurately examine the lesions in the natural cavities. In the prior art, when examining the lesion region of the cavity, it is usually necessary to first extend the hysteroscope into the cavity for observation. Once the lesion is found, the hysteroscope is kept and fixed into the body of the patient, and then a therapeutic instrument is extended into the body of the patient through the instrument operation hole provided in the hysteroscope for the treatment of the patient, such as shearing, cutting, coagulation operations.

The existing hysteroscope has only a single instrument operation hole. The instrument operation hole generally allows only a single instrument to pass through and use. Another instrument can be introduced into the instrument operation hole for the next operation only after the operation of the previous instrument is finished and the instrument is taken out from the instrument operation hole, so that the surgical efficiency is reduced, and the tissue cannot be cut and distracted at the same time (to separate and expose the incision boundary) for the operation that needs to expose the incision boundary, leading to the increase of the surgical difficulty.

### SUMMARY

An objective of the present disclosure is to provide a dual-channel instrument guiding device for hysteroscope . The dual-channel instrument guiding device for hysteroscope is provided with multiple instrument channels, through which multiple instruments can be operated at a lesion area of the cavity at the same time, so as to solve the problem that the surgical difficulty is increased as the tissue cannot be cut and distracted at the same time (to separate and expose the incision boundary) by means of the existing hysteroscope.

To achieve the objective above, the present disclosure employs the following technical solution:
A dual-channel instrument guiding device for hysteroscope provided by the present disclosure includes:
an operation sleeve, wherein one end of the operation sleeve is open, an other end of the operation sleeve is closed by a closure structure, and the closure structure is provided with a port for a hysteroscopic lens to pass through; and
a cavity dividing piece, wherein the cavity dividing piece is disposed in the operation sleeve and divides an interior of the operation sleeve into three channels isolated from one another, each of the channels extends in an axial direction of the operation sleeve, one of the channels is used as a lens channel for the hysteroscopic lens to pass through, and remaining two of the channels are used as instrument channels for surgical instruments to pass through.

Alternatively, a diameter of the operation sleeve is from 6 mm to 9 mm.

Alternatively, the cavity dividing piece includes:
a cylindrical guide tube, wherein the cylindrical guide tube is sleeved with the operation sleeve and arranged in the axial direction of the operation sleeve, both ends of the cylindrical guide tube are open, and the lens channel is arranged in the cylindrical guide tube; the cylindrical guide tube and the operation sleeve are tangental and connected at a tangent point, and an annular cavity channel is formed between an outer wall of the cylindrical guide tube and an inner wall of the operation sleeve; and
a cavity separating plate, wherein the cavity separating plate is disposed in the annular cavity channel and arranged in the axial direction of the operation sleeve, so as to separate the annular cavity channel into two instrument channels isolated from each other in a circumferential direction of the annular cavity channel.

Alternatively, the cavity separating plate is a flat plate, and the two instrument channels are symmetrically distributed on both sides of the cavity separating plate.

Alternatively, the operation sleeve and the cavity dividing piece are integrally formed.

Alternatively, the outer wall of the cylindrical guide tube is detachably connected to the inner wall of the operation sleeve by a first connecting structure, the first connecting structure comprises a first slide groove and a first strip-shaped projection in sliding fit with the first slide groove, both the first slide groove and the first strip-shaped projection extend in the axial direction of the operation sleeve; and one of the first slide groove and the first strip-shaped projection is disposed on the outer wall of the cylindrical guide tube, and an other of the first slide groove and the first strip-shaped projection is disposed on the inner wall of the operation sleeve.

Alternatively, the cavity separating plate is detachably connected to the inner wall of the operation sleeve by a second connecting structure, the second connecting structure comprises a second slide groove and a second strip-shaped projection in sliding fit with the second slide groove, and both the second slide groove and the second strip-shaped projection extend in the axial direction of the operation sleeve; and one of the second slide groove and the second strip-shaped projection is disposed on the inner wall of the operation sleeve, and an other of the second slide groove and the second strip-shaped projection is disposed on the cavity separating plate;
the cavity separating plate is detachably connected to the outer wall of the cylindrical guide tube by a third connecting structure, the third connecting structure comprises a third slide groove and a third strip-shaped projection in sliding fit with the third slide groove, and both the third slide groove and the third strip-shaped projection extend in the axial direction of the operation sleeve; and one of the third slide groove and the third strip-shaped projection is disposed on the outer wall of the cylindrical guide tube, and an other of the third slide groove and the third strip-shaped projection is disposed on the cavity separating plate.

Alternatively, the outer wall, close to the other end that is closed, of the operation sleeve is provided with two instrument inlets, and the two instrument inlets are communicated with the two instrument channels, respectively.

Alternatively, the outer wall, close to the other end that is closed, of the operation sleeve is provided with a water inlet connector and a water return connector, and the water inlet connector and the water return connector are communicated with the two instrument channels, respectively.

Alternatively, the water inlet connector and the water return connector are provided with a water inlet valve and a water return valve, respectively.

Alternatively, the closure structure is an elastic closure structure, and the port provided in the closure structure is a cut.

Alternatively, each of the operation sleeve and the cavity dividing piece is made of medical stainless steel, and the closure structure is made of rubber.

Compared with the prior art, the present disclosure has the following technical effects:
The dual-channel instrument guiding device for hysteroscope provided by the present disclosure is novel and reasonable in structure. The dual-channel instrument guiding device for hysteroscope is provided with a cavity dividing piece disposed in an operation sleeve thereof, the cavity dividing piece divides the interior of the operation sleeve into three channels isolated from one another. One of the channels is used as a lens channel for a hysteroscopic lens to pass through, and the remaining two of the channels are used as instrument channels for surgical instruments to pass through. During actual use, different instruments can be introduced into the two instrument channels simultaneously to operate on a lesion area in a human body cavity, which can cooperate with each other without interfering with each other. For example, a grasping instrument and a cutting instrument can be respectively introduced to different instrument channels to carry out tissue distraction and tissue cut simultaneously on the lesion area (to separate the tissue and expose the incision boundary), thus solving the problem that the existing hysteroscope cannot achieve the simultaneous operation of multiple instruments at the lesion area of the cavity, and avoiding the problems that the duration of surgery is prolonged and the surgical risk is increased as the instruments reach the lesion one after another, and the latter instrument needs to find the wound again. The dual-channel instrument guiding device for hysteroscope is provided with multiple channels, which can improve the surgical efficiency and reduce surgical risk while reducing the surgical difficulty.

The existing hysteroscopes are mostly of integrated structure, and a lens tube is inconvenient to disassemble, leading to the existence of disinfection dead angle. Based on this, in some technical solutions disclosed by the present disclosure, the cavity separating plate and the cylindrical guide tube are both detachably disposed in the operation sleeve. When cleaning and disinfection are required, the cavity separating plate, the cylindrical guide tube and the operation sleeve can be detached, and cleaned and disinfected, respectively, thus avoiding the problems of disinfection dead angle and incomplete cleaning.

In addition, in order to observe the cavity better, it is necessary to inject pressurized liquid, such as physiological saline, into the cavity to expand the cavity or flush the field of vision, facilitating examination, observation and surgical operation. Therefore, in addition to the instrument operation hole, a liquid channel is often required in hysteroscopy. In the existing hysteroscope, the liquid channel and the instrument operation hole are independently arranged, the liquid channel occupies an internal space of the hysteroscope, thus further limiting the number and diameter of instrument operation holes in the hysteroscope. Based on this, the two instrument channels in the present disclosure are provided with a water inlet connector and a water outlet connector, respectively, and thus each instrument channel not only can guide the instrument, but also serve as a circulation channel for liquids, such as physiological saline, present in the surgery, thus achieving the integration of the instrument channel and the liquid channel. The multi-instrument operation can be realized, a caliber of the instrument channel is increased, and an optional range of the instrument is enlarged. The pressurized liquid, such as physiological saline, can be injected into the cavity through the instrument channels. The device has novel structure and strong practicability.

### BRIEF DESCRIPTION OF THE DRAWINGS

To describe the technical solutions of the embodiments of the present disclosure or in the prior art more clearly, the following briefly introduces the accompanying drawings required for describing the embodiments. Apparently, the accompanying drawings in the following description show merely some embodiments of the present disclosure, and those of ordinary skill in the art may still derive other drawings from these accompanying drawings without creative efforts.
FIG. 1 is a front view of a dual-channel instrument guiding device for hysteroscope according to an embodiment of the present disclosure;
FIG. 2 is a structural schematic diagram of a proximal end (closed end) structure of the dual-channel instrument guiding device for hysteroscope according to an embodiment of the present disclosure;
FIG. 3 is a structural schematic diagram of a distal end (open end) structure of the dual-channel instrument guiding device for hysteroscope according to an embodiment of the present disclosure.

Reference numerals in the accompanying drawings:
100 dual-channel instrument guiding device for hysteroscope ;
1 operation sleeve; 2 closure structure; 21 port; 3 cylindrical guide tube; 31 lens channel; 4 cavity separating plate; 41 instrument channel; 5 first connecting structure; 6 second connecting structure; 7 third connecting structure; 8 instrument inlet; 9 water inlet connector; 10 water return connector.

### DETAILED DESCRIPTION OF THE EMBODIMENTS

The following clearly and completely describes the technical solutions in the embodiments of the present disclosure with reference to the accompanying drawings in the embodiments of the present disclosure. Apparently, the described embodiments are merely a part rather than all of the embodiments of the present disclosure. All other embodiments obtained by persons of ordinary skill in the art based on the embodiments of the present disclosure without creative efforts shall fall within the protection scope of the present disclosure.

An objective of the present disclosure is to provide a dual-channel instrument guiding device for hysteroscope . The dual-channel instrument guiding device for hysteroscope is provided with multiple operation channels, through which multiple instruments can be operated at a lesion region of the cavity at the same time, so as to solve the problem that the surgical difficulty is increased as the tissue cannot be cut and distracted at the same time (to expose the incision boundary) by means of the existing hysteroscope.

In order to make the objectives, technical solutions and advantages of the present disclosure more clearly, the present disclosure is further described in detail below with reference to the embodiments.

### Embodiment I

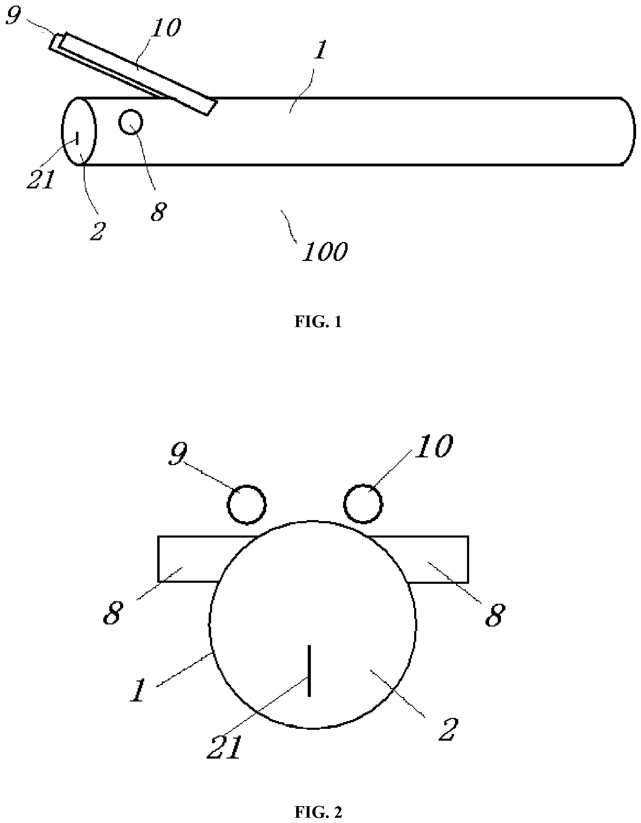

As shown in FIG. 1 to FIG. 3, a dual-channel instrument guiding device for hysteroscope 100 is provided by this embodiment, including an operation sleeve 1, and a cavity dividing piece. One end of the operation sleeve 1 is open, and the other end of the operation sleeve is closed by a closure structure 2. In a general case, the operation sleeve 1 is in a cylindrical shape, and the open end of the operation sleeve is often used to be inserted into a human body cavity, and is far away from the doctor, so the open end is also called "distal end". Correspondingly, the closed end of the operation sleeve is often located outside the human body and close to the doctor, so the closed end is also called "proximal end". The closure structure 2 is not absolutely closed, and is provided with a port 21 for a hysteroscopic lens or instrument to pass through. The cavity dividing piece is disposed in the operation sleeve 1 to divide the interior of the operation sleeve 1 into three channels isolated from one another. Each of the three channels extends in an axial direction of the operation sleeve 1, one of the channels is used as a lens channel 31 for the hysteroscopic lens to pass through, and the remaining two channels are used as instrument channels 41 for surgical instruments to pass through. In actual application, considering that the volume of the operation sleeve 1 should not be too large, a space of each instrument channel 41 only allows one surgical instrument to pass through. An overall outer diameter of the operation sleeve 1 is preferably from 6 mm to 9 mm.

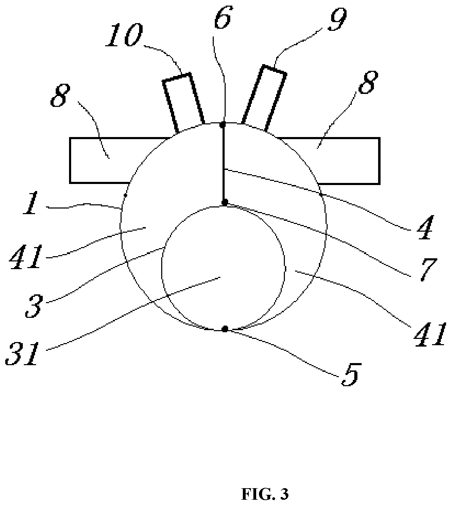

In this embodiment, the cavity dividing piece may be in various forms, which can be composed of multiple partition plates, or formed by combining the plate with components in other shape. As a preferred solution, the cavity dividing piece in this embodiment includes a cylindrical guide tube 3, and a cavity separating plate 4. As shown in FIG. 3, the cylindrical guide tube 3 is sleeved with the operation sleeve 1, and is arranged in the axial direction of the operation sleeve 1. Both ends of the cylindrical guide tube 3 are open for the lens to extend out or in, and respectively extend to both ends of the operation sleeve 1, and the interior of the cylindrical guide tube 3 is a lens channel 31 capable of being placed a lens. A diameter of the cylindrical guide tube 3 is less than that of the operation sleeve 1. The cylindrical guide tube 3 and the operation sleeve 1 may be arranged coaxially or internally tangentially. Considering that the space of the instrument channel 41 should be suitable for the corresponding surgical instrument 1 to pass through and should not be too small, the solution that the cylindrical guide tube 3 is in internally tangent with the operation sleeve 1 is preferably employed, an outer wall of the cylindrical guide tube 3 is connected to an inner wall of the operation sleeve 1 at a tangent point, and an annular cavity channel is further formed between the outer wall of the cylindrical guide tube 3 and the inner wall of the operation sleeve 1. The annular cavity channel is crescent-shaped as a whole, and a cavity separating plate 4 is detachably disposed in the annular cavity channel and arranged in the axial direction of the operation sleeve 1, so as to divide the annular cavity channel into two instrument channels 41 isolated from each other along the circumferential direction of the annular cavity channel.

Furthermore, in this embodiment, the cavity separating plate 4 is a flat plate, and only one cavity separating plate is provided in a general case. As shown in FIG. 3, the cavity separating plate 4 is located in diameter directions of the operation sleeve 1 and the cylindrical guide tube 3 simultaneously, and two instrument channels 41 formed by the separation of the cavity separating plate 4 are symmetrically distributed on both sides of the cavity separating plate 4, and the two instrument channels 41 have the completely same cross section shape and area. As shown in FIG. 3, a top end of the cavity separating plate 4 is fixedly connected to the inner wall of the operation sleeve 1 in a welding or integral forming manner, and a bottom end of the cavity separating plate 4 is detachably connected to the outer wall of the cylindrical guide tube 3; or the top end of the cavity separating cavity 4 is detachably connected to the inner wall of the operation sleeve 1, and the bottom end of the cavity separating cavity 4 is fixedly connected to the cylindrical guide tube 3 in a welding or integral forming manner; or the top end of the cavity separating plate 4 is detachably connected to the inner wall of the operation sleeve 1, and the bottom end of the cavity separating plate 4 is detachably connected to the cylindrical guide tube 3; or the cavity separating plate 4, the operation sleeve 1 and the cylindrical guide tube 3 are integrally formed.

Furthermore, in this embodiment, preferably, the outer wall of the cylindrical guide tube 3 are detachably connected to the inner wall of the operation sleeve 1 by a first connecting structure 5 at the tangent point. The first connecting structure 5 includes a first slide groove, and a first strip-shaped projection in sliding fit with the first slide groove, and both the first slide groove and the first strip-shaped projection extend in the axial direction of the operation sleeve 1. One of the first slide groove and the first strip-shaped projection is disposed on the outer wall of the cylindrical guide tube 3, and the other of the first slide groove and the first strip-shaped projection is disposed on the inner wall of the operation sleeve 1. As a preferred solution, the first slide groove is disposed on the inner wall of the operation sleeve 1 in a bonding, welding or integral forming manner, and extends in the axial direction of the operation sleeve 1, while the first strip-shaped projection is disposed on the outer wall of the cylindrical guide tube 3 in a bonding, a welding or an integral forming manner.

Furthermore, one end edge of the cavity separating plate 4 is detachably connected to the inner wall of the operation sleeve 1 by a second connecting structure 6. The second connecting structure 6 includes a second slide groove, and a second strip-shaped projection in sliding fit with the second slide groove, and both the second slide groove and the second strip-shaped projection extend in the axial direction of the operation sleeve 1. One of the second slide groove and the second strip-shaped projection is disposed on the inner wall of the operation sleeve 1, and the other of the second slide groove and the second strip-shaped projection is disposed on the cavity separating plate 4. As a preferred solution, the second slide groove is disposed on the inner wall of the operation sleeve 1 in a bonding, welding or integral forming manner, and is preferably arranged symmetrically with the first slide groove in an up and down direction. The second strip-shaped projection is disposed on the one end edge of the cavity separating plate 4 in a bonding, a welding or an integral forming manner. Correspondingly, the cavity separating plate 4 is detachably connected to the outer wall of the cylindrical guide tube 3 by a third connecting structure 7. The third connecting structure 7 includes a third slide groove, and a third strip-shaped projection in sliding fit with the third slide groove, and both the third slide groove and the third strip-shaped projection extend in the axial direction of the operation sleeve 1. One of the third slide groove and the third strip-shaped projection is disposed on the outer wall of the cylindrical guide tube 3, and the other of the third slide groove and the third strip-shaped projection is disposed on the cavity separating plate 4. As a preferred solution, the third slide groove is disposed on the outer wall of the cylindrical guide tube 3 in a bonding, welding or integral forming manner, and is preferably arranged symmetrically with the first slide groove in the up and down direction. The third strip-shaped projection is disposed on the cavity separating plate 4 in a bonding, a welding or an integral forming manner. The third strip-shaped projection and the second strip-shaped on the cavity separating plate 4 are symmetrically arranged in the up and down direction.

Based on sliding connecting structures of the first connecting structure 5, the second connecting structure 6 and the third connecting structure 7, both the cavity separating plate 4 and the cylindrical guide tube 3 can be detached from the operation sleeve 1, and the cavity separating plate 4 and the cylindrical guide tube 3 may also be detached from each other. During installation, the installation can be achieved by means of the sliding fit between the strip-shaped projection and the corresponding slide groove, and the installation is simple and convenient.

In this embodiment, the outer wall, close to the closed end, of the operation sleeve 1 is provided with two instrument inlets 8, as shown in FIG. 2 and FIG. 3. The two instrument inlets 8 are communicated with two instrument channels 41, respectively, and are located on both sides of the operation sleeve 1, respectively.

In this embodiment, the outer wall, close to the closed end, of the operation sleeve 1 is further provided with a water inlet connector 9 and a water return connector 10, and the water inlet connector 9 and the water return connector 10 are communicated with different instrument channels 41, respectively. As shown in FIG. 2 and FIG. 3, two instrument channels 41 are provided, the water inlet connector 9 and the water return connector 10 are communicated with the two instrument channels 41, respectively. The instrument inlets 8 are located in front of the water inlet connector 9 and the water return connector 10. That is, the instrument inlets 8 are closer to the closed end of the operation sleeve 1 than the water inlet connector 9 and the water return connector 10, and need to be exposed outside the body of the patient in the actual operation. The water inlet connector 9 and the water return connector 10 are both preferably in a tubular structure, are arranged in an inclined manner, and are angled to the axial direction of the operation sleeve 1. In a general case, outer ends of the water inlet connector 9 and the water return connector are both inclined towards the closed end of the operation sleeve 11, as shown in FIG. 1 to FIG. 3. Each instrument channel 41 not only can guide instruments, but also can serve as a circulation channel for liquids, such as physiological saline, presented in operation, thus achieving the integration of the instrument channel and the liquid channel, so that the multi-instrument operation can be realized, and pressurized liquid, such as physiological saline, can be injected into the cavity through the instrument channels if necessary. The dual-channel instrument guiding device for hysteroscope has novel structure and strong practicability.

In this embodiment, the water inlet connector 9 and the water return connector 10 are respectively used for injecting liquid (e.g., physiological saline) into the human body cavity and guiding out the liquid in the human body cavity. In order to control the injection and guiding-out rate of the liquid (e.g., physiological saline), a water inlet valve and a water return valve can be arranged on the water inlet connector 9 and water return connector 10, respectively. The water inlet valve and the water return valve are preferably solenoid valves.

In this embodiment, the closure structure 2 is an elastic closure structure, which can be fixed to the operation sleeve 1 in a bonding manner. A port 21 formed in the closure structure 2 is a cut (a cutting gap), such as a straight cut, a cross cut or a Union jack-shaped cut. As the closure structure 2 is an elastic structure, the cut not only can allow the lens to pass through, but also can keep the cut closed by means of the elasticity of the material of the closure structure 2, so as to prevent the liquid from leaking through the port 21 when there is liquid circulating in the instrument channels 41.

In this embodiment, the closure structure 2 and the operation sleeve 1 are made of rubber. The first connecting structure 5, the second connecting structure 6, the third connecting structure 7, the cavity separating cavity 4 and the cylindrical guide tube 3 are preferably made of the same rubber.

During actual use, the operation sleeve 1 is held by a doctor, and the hysteroscopic lens can be inserted through the cylindrical guide tube 3 to collect an image. By means of the water inlet connector 9 and the water return connector 10, the flush of the lesions in the cavity by the normal saline and the pressure adjustment of the normal saline can be achieved to expand the cavity. Two medical instruments can be inserted through the two instrument inlets 8 and can assist each other to carry out therapeutic operation, which greatly improves the surgical convenience and operability of the doctor, shortens the duration of surgery, reducing the surgical risk. After the operation is finished, the cylindrical guide tube 3 and the cavity separating plate 4 both can be detached for disinfection and maintenance, which not only eliminates the disinfection dead angle, but also is conducive to independent protection of the lens.

It should be noted that it is apparent to those skilled in the art that the present disclosure is not limited to the details of the above exemplary embodiments, and can be realized in other specific forms without departing from the spirit or basic characteristics of the present disclosure. Therefore, the embodiments should be considered as exemplary and non-limiting in all aspects, and the scope of the present disclosure is defined by the appended claims rather than the above description, so it is intended to embrace all changes that fall within the meaning and range of equivalents of the claims, and any reference signs in the claims should not be regarded as limiting the claims involved.

Specific examples are used herein for illustration of the principles and implementation methods of the present disclosure. The description of the embodiments is merely used to help illustrate the method and its core principles of the present disclosure. In addition, a person of ordinary skill in the art can make various modifications in terms of specific embodiments and scope of application in accordance with the teachings of the present disclosure. In conclusion, the content of this specification shall not be construed as a limitation to the present disclosure.

## Claims

1. A dual-channel instrument guiding device for hysteroscope, comprising:
an operation sleeve, wherein one end of the operation sleeve is open, an other end of the operation sleeve is closed by a closure structure, and the closure structure is provided with a port for a hysteroscopic lens to pass through; and
a cavity dividing piece, wherein the cavity dividing piece is disposed in the operation sleeve and divides an interior of the operation sleeve into three channels isolated from one another, each of the channels extends in an axial direction of the operation sleeve, one of the channels is used as a lens channel for the hysteroscopic lens to pass through, and remaining two of the channels are used as instrument channels for surgical instruments to pass through.

2. The dual-channel instrument guiding device for hysteroscope according to claim 1, wherein the cavity diving piece comprises:
a cylindrical guide tube, wherein the cylindrical guide tube is sleeved with the operation sleeve and arranged in the axial direction of the operation sleeve, both ends of the cylindrical guide tube are open, and the lens channel is arranged in the cylindrical guide tube; the cylindrical guide tube and the operation sleeve are tangental and connected at a tangent point, and an annular cavity channel is formed between an outer wall of the cylindrical guide tube and an inner wall of the operation sleeve; and
a cavity separating plate, wherein the cavity separating plate is disposed in the annular cavity channel and arranged in the axial direction of the operation sleeve, so as to separate the annular cavity channel into two instrument channels isolated from each other in a circumferential direction of the annular cavity channel.

3. The dual-channel instrument guiding device for hysteroscope according to claim 2, wherein the cavity separating plate is a flat plate, and the two instrument channels are symmetrically distributed on both sides of the cavity separating plate.

4. The dual-channel instrument guiding device for hysteroscope according to any one of claims 1 to 3, wherein the operation sleeve and the cavity dividing piece are integrally formed.

5. The dual-channel instrument guiding device for hysteroscope according to claim 2 or 3, wherein the outer wall of the cylindrical guide tube is detachably connected to the inner wall of the operation sleeve by a first connecting structure, the first connecting structure comprises a first slide groove and a first strip-shaped projection in sliding fit with the first slide groove, both the first slide groove and the first strip-shaped projection extend in the axial direction of the operation sleeve; and one of the first slide groove and the first strip-shaped projection is disposed on the outer wall of the cylindrical guide tube, and an other of the first slide groove and the first strip-shaped projection is disposed on the inner wall of the operation sleeve.

6. The dual-channel instrument guiding device for hysteroscope according to claim 2 or 3, wherein the cavity separating plate is detachably connected to the inner wall of the operation sleeve by a second connecting structure, the second connecting structure comprises a second slide groove and a second strip-shaped projection in sliding fit with the second slide groove, and both the second slide groove and the second strip-shaped projection extend in the axial direction of the operation sleeve; and one of the second slide groove and the second strip-shaped projection is disposed on the inner wall of the operation sleeve, and an other of the second slide groove and the second strip-shaped projection is disposed on the cavity separating plate;
the cavity separating plate is detachably connected to the outer wall of the cylindrical guide tube by a third connecting structure, the third connecting structure comprises a third slide groove and a third strip-shaped projection in sliding fit with the third slide groove, and both the third slide groove and the third strip-shaped projection extend in the axial direction of the operation sleeve; and one of the third slide groove and the third strip-shaped projection is disposed on the outer wall of the cylindrical guide tube, and an other of the third slide groove and the third strip-shaped projection is disposed on the cavity separating plate.

7. The dual-channel instrument guiding device for hysteroscope according to any one of claims 1 to 3, wherein the outer wall, close to the other end that is closed, of the operation sleeve is provided with two instrument inlets, and the two instrument inlets are communicated with the two instrument channels, respectively.

8. The dual-channel instrument guiding device for hysteroscope according to any one of claims 1 to 3, wherein the outer wall, close to the other end that is closed, of the operation sleeve is provided with a water inlet connector and a water return connector, and the water inlet connector and the water return connector are communicated with the two instrument channels, respectively.

9. The dual-channel instrument guiding device for hysteroscope according to claim 8, wherein the water inlet connector and the water return connector are provided with a water inlet valve and a water return valve, respectively.

10. The dual-channel instrument guiding device for hysteroscope according to any one of claims 1 to 4, wherein the closure structure and the operation sleeve are integrally formed, the closure structure is an elastic closure structure, and the port provided in the closure structure is a cut.
